# EUROPÄISCHE PATENTSCHRIFT

(11) **EP 2 393 434 B1**
(45) Veröffentlichungstag und Bekanntmachung des Hinweises auf die Patenterteilung: **27.01.2016**
(21) Anmeldenummer: 10701542.2
(22) Anmeldetag: 29.01.2010
(51) Int. Cl.: A61B 17/16, A61B 17/28, A61B 17/295

(54) **CHIRURGISCHES SCHIEBESCHAFTINSTRUMENT UND SCHIEBESCHAFT**
SURGICAL SLIDING SHAFT INSTRUMENT AND SLIDING SHAFT
INSTRUMENT CHIRURGICAL À TIGE COULISSANTE ET TIGE COULISSANTE

(30) Priorität: 06.02.2009 DE 102009008719
(43) Veröffentlichungstag der Anmeldung: 14.12.2011
(73) Patentinhaber: Aesculap AG, 78532 Tuttlingen (DE)
(72) Erfinder: WEIßHAUPT, Dieter, 78194 Immendingen (DE); FAULHABER, Konstantin, Frittlingen 78665 (DE); SCHULZ, Peter, Löffingen 79843 (DE); NESPER, Markus, Tuttlingen 78532 (DE)
(74) Vertreter: Hoeger, Stellrecht & Partner Patentanwälte mbB
(86) Internationale Anmeldenummer: PCT/EP2010/051058
(87) Internationale Veröffentlichungsnummer: WO 2010/089249

(56) Entgegenhaltungen:
- EP-A1- 1 967 145
- US-A- 5 385 570
- US-A1- 2004 186 499
- US-A1- 2005 090 829

## Beschreibung

Die vorliegende Erfindung betrifft einen Schiebeschaft für ein chirurgisches Schiebeschaftinstrument, welcher eine Längsrichtung definiert, mit einem ersten Schaftteil und einem zweiten Schaftteil, die relativ zueinander verschiebbar angeordnet sind, welcher erste Schaftteil ein erstes Werkzeugelement trägt und welcher zweite Schaftteil ein zweites Werkzeugelement trägt, wobei der zweite Schaftteil eine Schneidgutaufnahme aufweist mit einer Aufnahmeöffnung, welche vom zweiten Werkzeugelement begrenzt ist und sich ausgehend von diesem in proximaler Richtung erstreckt, wobei der zweite Schaftteil mindestens zweiteilig ausgebildet ist und ein Werkzeugteil, welches das zweite Werkzeugelement umfasst, und ein mit dem Werkzeugteil verbundenes Schiebeteil umfasst.

Ferner betrifft die vorliegende Erfindung ein chirurgisches Schiebeschaftinstrument umfassend einen Instrumentengriff und einen mittels des Instrumentengriffs betätigbaren Schiebeschaft.

Chirurgische Schiebeschaftinstrumente der eingangs beschriebenen Art sind beispielsweise in Form von Knochenstanzen bekannt wie sie in der US 2004/0186499 A1 und der US 5,385,570 beschrieben sind. Sie sind derart ausgebildet, dass die beiden den Schiebeschaft ausbildenden Schaftteile direkt aneinander geführt sind. Hierfür sind sogenannte innenliegende Führungselemente zur Ausbildung der Führungseinrichtung vorgesehen, die an den aneinander anliegenden Flächen der Schaftteile angeordnet beziehungsweise ausgebildet sind. Die beiden Schaftteile, die auch als Schieberoberteil und Schieberunterteil bezeichnet werden, liegen direkt aneinander an. Dies hat jedoch den Nachteil, dass zwischen die aneinander anliegenden Schaftteile bei der Reinigung oder auch bei einer Verwendung des Instruments Flüssigkeit hineingesaugt werden kann. Diese zu entfernen ist praktisch nicht möglich, ohne das Instrument zu zerlegen.

Es ist daher eine Aufgabe der vorliegenden Erfindung, einen Schiebeschaft und ein chirurgisches Schiebeschaftinstrument der eingangs beschriebenen Art so zu verbessern, dass sie auf einfache Weise herstellbar sind.

Diese Aufgabe wird bei einem Schiebeschaft der eingangs beschriebenen Art erfindungsgemäß dadurch gelöst, dass das Werkzeugteil und das Schiebeteil unlösbar miteinander verbunden sind und dass das Werkzeugteil und das Schiebeteil durch Schweißen, Löten oder Kleben miteinander verbunden sind oder dass das Schiebeteil durch Anspritzen an das Werkzeugteil hergestellt ist.

Die mindestens zweiteilige Ausbildung des zweiten Schaftteils ermöglicht es insbesondere, eine Schneidgutaufnahme, deren Innenquerschnitt in proximaler Richtung hin zunimmt, auf einfache Weise herzustellen. Ferner bietet die zweiteilige Ausbildung die Option, den zweiten Schaftteil aus zwei unterschiedlichen Materialien herzustellen. Da es sich insbesondere beim zweiten Werkzeugelement des zweiten Schaftteils um ein Verschleißteil handelt, können durch den zweiteiligen Aufbau bei der Herstellung Kosten gespart werden, da nur das Werkzeugteil aus einem Material hergestellt werden muss, welches die Erfordernisse an das zweite Werkzeugelement erfüllt, nicht jedoch das Schiebeteil. Die Handhabung sowie die Sicherheit des Schiebeschafts werden auf einfache Weise dadurch verbessert werden, dass das Werkzeugteil und das Schiebeteil unlösbar miteinander verbunden sind. Das Werkzeugteil und das Schiebeteil bilden dann, wie bei herkömmlichen Schiebeschäften, den zweiten Schaftteil aus, welcher für den Anwender eine Einheit bildet. Vorteilhafterweise sind das Werkzeugteil und das Schiebeteil durch Schweißen, Löten oder Kleben miteinander verbunden. Die beschriebenen Verbindungsarten eignen sich hervorragend, um das Werkzeugteil und das Schiebeteil unlösbar miteinander zu verbinden und eine den zweiten Schaftteil ausbildende Einheit zu schaffen.

Vorteilhaft ist es, dass das Schiebeteil durch Anspritzen an das Werkzeugteil hergestellt ist. Insbesondere dann, wenn das Werkzeugteil aus einem Metall hergestellt ist, kann so das Schiebeteil in einem zweiten Schritt durch Anspritzen an das Werkzeugteil mit diesem verbunden werden. Das Schiebeteilmaterial kann dabei insbesondere ein Kunststoff oder aber auch ein Metall sein.

Vorteilhaft ist es, wenn das mindestens eine erste und/oder zweite Führungselement in einer Richtung vom zweiten Schaftteil weg weisend und/oder seitlich geöffnet ist. Dies ermöglicht es, dass die Führungseinrichtung einfach und sicher gereinigt werden kann. Durch beispielsweise eine vorgesehene seitliche Öffnung kann die Führungseinrichtung mittels einer Spülflüssigkeit einfach und sicher von Verunreinigungen befreit werden.

Günstig kann es ferner sein, insbesondere auch bei einem Schiebeschaft der eingangs beschriebenen Art, wenn der erste Schaftteil und der zweite Schaftteil nur in einem Führungsbereich, in welchem die ersten und die zweiten Führungselemente ineinandergreifen, aneinander anliegen und abgesehen vom Führungsbereich durch einen Reinigungsspalt voneinander beabstandet sind. Der erfindungsgemäß vorgesehene Reinigungsspalt hat den Vorteil, dass Verunreinigungen, die in den Bereich zwischen die beiden Schaftteile gelangen, ohne Weiteres durch Spülen oder Waschen des Instruments in einer Waschmaschine wieder beseitigt werden können. Der Reinigungsspalt verhindert also, dass Flüssigkeiten zwischen die herkömmlicherweise aneinander anliegende Schaftteile hineingesaugt werden können. Das Instrument braucht daher für eine umfassende Reinigung und Sterilisierung nicht zerlegt werden. Der Reinigungsspalt ist vorzugsweise so groß, dass er einfach mit einer Reinigungsflüssigkeit durchgespült werden kann.

Günstigerweise weist der Reinigungsspalt eine Größe auf, insbesondere eine Breite, welche ausreicht, um das Auftreten einer Kapillarwirkung für eine Reinigungsflüssigkeit zu vermeiden. Eine Breite des Reinigungsspalts liegt vorzugsweise in einem Bereich zwischen 0,2 mm und 2 mm. Günstig ist es, wenn der Spalt eine Breite in einem Bereich von 0,2 mm bis 1 mm, vorzugsweise 0,6 mm, aufweist. Zur Ausbildung des Reinigungsspalts können das erste und/oder das zweite Schaftteil mit einer entsprechenden Ausnehmung oder Vertiefung versehen sein. Die Herstellung wird besonders einfach, wenn die Ausnehmung, deren Tiefe vorzugsweise die Breite des Reinigungsspalts definiert, nur an einem Schaftteil vorgesehen ist. Vorzugsweise ist dies durch eine Ausnehmung am zweiten Schaftteil realisiert. Vorzugsweise erstreckt sich die Ausnehmung ausgehend vom Führungsbereich in proximaler Richtung, optional bis zu einem proximalen Ende des zweiten Schaftteils.

Vorteilhaft ist es, wenn das mindestens eine erste oder zweite Führungselement in Form einer Führungsnut ausgebildet ist und wenn das korrespondierende andere Führungselement in Form eines formschlüssig in die Führungsnut eingreifenden Führungsvorsprungs ausgebildet ist. Durch die formschlüssig zusammenwirkenden Führungselemente in Form der Führungsnut und des Führungsvorsprungs kann eine gute und verkantungsfreie Führung der beiden Schaftteile aneinander realisiert werden.

Um auf einfache Weise eine definierte Bewegung parallel zu einer vom Schiebeschaft definierten Längsachse zu erreichen, ist es günstig, wenn sich das mindestens eine erste oder zweite Führungselement parallel zu einer vom Schiebeschaft definierten Längsachse über einen Bereich erstreckt, welcher mindestens einem relativen Verschiebeweg zwischen dem ersten und zweiten Schaftteil entspricht. So kann eine Führung der Schaftteile aneinander über den gesamten gewünschten Bewegungsweg sichergestellt werden.

Damit eine Beweglichkeit der Schaftteile relativ zueinander über einen gewünschten Bewegungsweg sichergestellt werden kann, ist es vorteilhaft, wenn sich das andere der Führungselemente parallel zu einer vom Schiebeschaft definierten Längsachse über einen Bereich erstreckt, welcher kleiner ist als der Führungsbereich. Durch entsprechende Wahl der Erstreckungen der ersten und zweiten Führungselemente kann so auf einfache Weise ein Bewegungsweg oder Hub der beiden Schaftteile relativ zueinander vorgegeben werden.

Um auf einfache Weise eine formschlüssige Verbindung zwischen den Führungselementen der Führungseinrichtung herstellen zu können, ist es günstig, wenn das mindestens eine erste oder das mindestens eine zweite Führungselement mindestens einseitig hinterschnitten ausgebildet ist. So kann zudem sichergestellt werden, dass die Schaftteile nicht in einer Richtung senkrecht zur Längsachse des Schiebeschafts voneinander getrennt werden können, wenn die beiden Führungselemente miteinander in Eingriff stehen.

Vorteilhafterweise nimmt eine Breite der Führungsnut quer zur von der Führungseinrichtung definierten Bewegungsrichtung in Richtung auf das zweite Schaftteil hin ab. Die Führungsnut kann so insbesondere in Form einer Schwalbenschwanznut ausgebildet werden. Denkbar wäre jedoch auch die Ausbildung einer im Querschnitt T-förmigen Nut.

Besonders einfach wird der Aufbau des Schiebeschafts, wenn nur ein einziges erstes und/oder ein einziges zweites Führungselement vorgesehen sind.

Vorteilhaft ist es, wenn die einzigen ersten und/oder zweiten Führungselemente im Bereich distaler Enden des ersten und zweiten Schaftteils angeordnet sind. Insbesondere dann, wenn die Werkzeugelemente im Bereich der distalen Enden angeordnet sind, kann so eine optimale Führung derselben bei deren Zusammenwirken ermöglicht werden.

Günstigerweise, insbesondere auch bei einem Schiebeschaft der eingangs beschriebenen Art, weist die Führungseinrichtung mindestens eine Spülöffnung auf zum Herstellen einer Fluidverbindung zwischen einem von der Führungseinrichtung definierten Führungsraum und einer Umgebung des Instruments. Durch die Spülöffnung hindurch kann eine Reinigungs- oder Spülflüssigkeit in den Führungsraum eingeleitet und auch wieder aus diesem herausgeleitet werden. Es können zwei, drei oder noch mehr Spülöffnungen vorgesehen sein, um die Führungseinrichtung einfach und sicher reinigen zu können.

Der Aufbau des Schiebeschaftinstruments vereinfacht sich weiter, wenn der Führungsraum durch einen Innenraum der Führungsnut definiert wird. Der Führungsraum wird in einem solchen Fall insbesondere begrenzt durch Seitenwände der Nut sowie einen Nutboden, welcher einem durch die Führungsnut definierten Schlitz gegenüberliegt.

Besonders einfach herstellen lässt sich die Führungseinrichtung, wenn die mindestens eine Spülöffnung in Form einer Durchbrechung einer die Führungsnut begrenzenden Seitenwand des ersten Schaftteils ausgebildet ist. Beispielsweise bei einer rechteckigen oder schwalbenschwanzförmigen Führungsnut stehen insgesamt drei Seitenwände zur Verfügung, die mit einer oder mehreren Spülöffnungen ausgestattet sein können.

Die Herstellung des Schiebeschaftinstruments vereinfacht sich weiter, wenn die Durchbrechung in Form eines Schlitzes oder eines Langlochs ausgebildet ist.

Um den zweiten Schaftteil individuell ausbilden zu können, ist es vorteilhaft wenn das Schiebeteil aus einem Schiebeteilmaterial und wenn das Werkzeugteil aus einem Werkzeugteilmaterial hergestellt ist. Es kann so das für jeden Einsatzzweck optimale Material für die jeweiligen Teile des zweiten Schaftteils ausgewählt werden.

Günstig ist es, wenn das Schiebeteilmaterial und das Werkzeugteilmaterial unterschiedliche Materialien sind. Dies gestattet es, bei der Auswahl der Materialien zur Ausbildung der beiden Teile die jeweils besonderen Erfordernisse an die Teile zu berücksichtigen.

Vorteilhafterweise ist das Werkzeugteilmaterial ein Metall. Insbesondere ist das Werkzeugmaterial ein Instrumentenstahl. Ein Metall, insbesondere Instrumentenstahl, eigenen sich hervorragend zur Ausbildung eines zweiten Werkzeugelements in Form einer Schneide, welche durch Schleifen so geschärft werden kann, dass mit ihr Knochen in Verbindung mit dem ersten Werkzeugelement ausstanzbar ist.

Die Stabilität des Schiebeschafts kann auf einfache Weise dadurch erhöht werden, dass das Schiebeteilmaterial ein Metall ist. Insbesondere kann es sich bei dem Metall um einen Instrumentenstahl handeln.

Die Herstellungskosten des Schiebeschafts lassen sich deutlich reduzieren, wenn das Schiebeteilmaterial ein Kunststoff ist. Insbesondere kann so die Herstellung des Schiebeschafts vereinfacht werden. Beispielsweise kann das Scheibeteil aus dem Kunststoff durch Spritzen hergestellt werden. Durch die teilweise Herstellung des zweiten Schaftteils aus einem Kunststoff eignet es sich insbesondere als Einwegartikel. Wird beispielsweise das zweite Werkzeugelement beschädigt oder unbrauchbar, beispielsweise eine Schneide stumpf, dann muss vom Schiebeschaft lediglich der zweite Schaftteil entfernt und ausgetauscht werden, der erste Schaftteil kann weiter genutzt werden. Dies reduziert Anschaffungs- und insbesondere auch Wiederaufbereitungskosten für Anwender des Schiebeschafts.

Um das Verbinden des Werkzeugteils und des Schiebeteils zu erleichtern, ist es günstig, wenn das Werkzeugteil mindestens einen mindestens teilweise umspritzbaren, in proximaler Richtung abstehenden Verbindungsvorsprung aufweist. Das Schiebeteil kann so auf einfache Weise durch Umspritzen des Verbindungsvorsprungs an das Werkzeugteil angeformt und unlösbar mit dem Werkzeugteil verbunden werden.

Die Reinigbarkeit des Schiebeschafts kann auf einfache Weise dadurch weiter verbessert werden, am Schiebeteil mindestens eine Spüldurchbrechung ausgebildet ist.

Vorteilhaft ist es, wenn das erste Werkzeugelement und das zweite Werkzeugelement zusammen ein Stanzwerkzeug ausbilden und wenn die Arbeitsstellung, in welcher das erste und das zweite Werkzeugelement aneinander anliegen und zusammenwirken, eine Stanzstellung definiert. Diese Ausgestaltung gestattet es, beispielsweise Knochen oder Knorpel in der Stanzstellung mittels des Schiebeschaftinstruments in gewünschter Weise zu entfernen.

Die eingangs gestellte Aufgabe wird ferner bei einem chirurgischen Schiebeschaft der eingangs beschriebenen Art erfindungsgemäß dadurch gelöst, dass das mindestens eine erste und/oder zweite Führungselement in einer Richtung vom zweiten Schaftteil weg weisend und/oder seitlich geöffnet ist.

Die erfindungsgemäß vorgeschlagene Lösung ermöglicht es, dass die Führungseinrichtung des Schiebeschafts einfach und sicher gereinigt werden kann. Durch beispielsweise eine vorgesehene seitliche Öffnung kann die Führungseinrichtung mittels einer Spülflüssigkeit einfach und sicher von Verunreinigungen befreit werden.

Vorteilhafterweise umfasst das Schiebeschaftinstrument einen der oben beschriebenen Schiebeschäfte. Das Schiebeschaftinstrument weist damit auch die oben im Zusammenhang mit den bevorzugten Ausführungsformen der Schiebeschäfte beschriebenen Vorteile auf.

Einfach und sicher kann ein Operateur das Schiebeschaftinstrument handhaben, wenn der Instrumentengriff in Form eines Griffteils an einem proximalen Ende des Schiebeschafts angeordnet oder ausgebildet ist.

Gemäß einer bevorzugten Ausführungsform der Erfindung kann vorgesehen sein, dass der Griffteil ein erstes und ein zweites Griffelement umfasst, welche relativ zueinander beweglich angeordnet sind, und dass das erste Griffelement mit dem ersten Schaftteil und dass das zweite Griffelement mit dem zweiten Schaftteil gekoppelt ist. Eine Relativbewegung der beiden Griffteile kann bei einer solchen Konstruktion auf einfache Weise eine Relativbewegung der beiden Schaftteile zueinander bewirken.

Der Aufbau des Schiebeschaftinstruments kann auf einfache Weise dadurch weiter vereinfacht werden, wenn das erste und das zweite Griffelement aneinander beweglich gelagert sind und einen Schlussbereich definieren. Unter einem Schlussbereich ist insbesondere der Bereich zu verstehen, in dem die Griffelemente aneinander gelagert sind, ähnlich wie zum Beispiel bei herkömmlichen Scheren.

Vorteilhaft ist es ferner, wenn der Schlussbereich am ersten Schaftteil und/oder am zweiten Schaftteil und/oder am ersten und/oder am zweiten Griffelement mindestens eine Schlussbereichausnehmung umfasst, welche in einer beliebigen Stellung der ersten und zweiten Schaftteile relativ zueinander frei zugänglich ist durch mindestens eine Schlussbereichsöffnung hindurch, welche eine ausreichende Größe aufweist, um eine Kapillarwirkung für eine Reinigungsflüssigkeit zu vermeiden. Die Schlussbereichsöffnung ermöglicht es auch, anders als bei bekannten Schiebeschaftinstrumenten, Verunreinigungen im Schlussbereich zu beseitigen, beispielsweise durch Spülen mit einer Reinigungs- oder Spülflüssigkeit. Die Schlussbereichsöffnung kann eine beliebige Querschnittsform aufweisen, insbesondere kann sie in Form einer Bohrung ausgebildet sein oder zum Beispiel einen eckigen Querschnitt aufweisen. Günstigerweise liegt ein minimaler Durchmesser beziehungsweise eine minimale Breite der Schaftbereichsöffnung in einem Bereich von 0,2 mm bis 10 mm.

Der Aufbau des Schiebeschaftinstruments kann weiter vereinfacht werden, wenn der erste Schaftteil und das erste Griffelement unbeweglich miteinander verbunden sind. Insbesondere können die beiden Teile auch einstückig ausgebildet sind, vorzugsweise aus einem Instrumentenstahl.

Betätigungskräfte lassen sich einfach und sicher vom zweiten Griffelement auf das zweite Schaftteil übertragen, wenn diese beweglich miteinander verbunden sind.

Ein besonders einfacher Aufbau des Schiebeschaftinstruments kann erreicht werden, wenn das zweite Griffelement verschwenkbar mit dem zweiten Schaltteil gekoppelt ist.

Günstig kann es auch sein, wenn das zweite Griffelement am Griffteil verschwenkbar gelagert ist. So kann insbesondere eine Lagerung des zweiten Griffelements unabhängig vom ersten Griffelement erreicht werden.

Bei einer weiteren bevorzugten Ausführungsform der Erfindung kann ferner eine Rückstelleinrichtung zum automatischen Rückführen des Schiebeschaftinstruments von einer aus einer Ruhestellung, in welcher ein Abstand zwischen dem ersten und dem zweiten Werkzeugelement maximal ist, ausgelenkten Stellung, in welcher ein Abstand zwischen dem ersten und dem zweiten Werkzeugelement kleiner ist als in der Ruhestellung, vorgesehen sein. Durch eine derartige Rückstelleinrichtung kann sichergestellt werden, dass die Werkzeugelemente voneinander beabstandet sind, wenn das Schiebeschaftinstrument unbetätigt ist. Es kann so von einer Bedienperson ergriffen werden und ist sofort bereit, um mit ihm durch Zusammenwirken der Werkzeugelemente in gewünschter Weise einen chirurgischen Eingriff vornehmen zu können.

Besonders einfach und kompakt aufbauen lässt sich das Schiebeschaftinstrument, wenn die Rückstelleinrichtung am Griffteil angeordnet ist oder angreift. Bei einer Zwangskopplung beispielsweise mindestens eines Griffelements des Griffteils mit mindestens einem Schaftteil kann so die Rückstelleinrichtung auf Grund eines Angreifens an diesem Griffelement eine Relativbewegung der Schaftteile in die Ruhestellung bewirken. Auf einfache Weise lässt sich die Rückstelleinrichtung ausbilden, wenn sie mindestens ein Rückstellglied umfasst. Denkbar ist es auch, zwei oder drei Rückstellglieder vorzusehen. Insbesondere können diese so angeordnet und/oder ausgebildet sein, dass sie direkt aneinander angreifen und zusammenwirken.

Günstig ist es, wenn die Rückstelleinrichtung mindestens ein vorspannendes Element umfasst, welches das Schiebeschaftinstrument in der Grundstellung hält.

Die Herstellung des Schiebeschaftinstruments vereinfacht sich weiter, wenn das mindestens eine vorspannende Element oder das mindestens eine Rückstellglied in Form eines Federelements ausgebildet sind. Das Federelement kann insbesondere in Form einer Schrauben- oder Blattfeder ausgebildet sein.

Günstig ist es, wenn das mindestens eine vorspannende Element auf einem Vorsprung des ersten und/oder zweiten Griffelements angeordnet ist. Insbesondere bei der Verwendung von Blattfedern kann so auf einfache Weise verhindert werden, dass das vorspannende Element direkt an dem ersten oder zweiten Griffelement anliegt, was den Nachteil hat, dass wiederum auf Grund einer Kapillarwirkung Flüssigkeit zwischen das Griffelement und das mindestens eine vorspannende Element gesaugt werden kann. Der Abstand zwischen dem vorspannenden Element und dem ersten und/oder zweiten Griffelement beträgt vorzugsweise mindestens 0,2 mm. Auf einfache Weise erreichen lässt sich ein solcher Abstand beispielsweise durch ein Abstandselement in Form eines Vorsprungs oder einer Unterlegscheibe.

Um einfach und sicher Knochenmaterial aus einem Knochen herausstanzen zu können, ist es günstig, wenn das Schiebeschaftinstrument in Form einer Knochenstanze ausgebildet ist.

Die nachfolgende Beschreibung bevorzugter Ausführungsformen der Erfindung dient im Zusammenhang mit der Zeichnung der näheren Erläuterung. Es zeigen:
- Figur 1:: eine teilweise geschnittene beziehungsweise durchbrochene Seitenansicht eines chirurgischen Schiebeschaftinstruments in Form einer Knochenstanze;

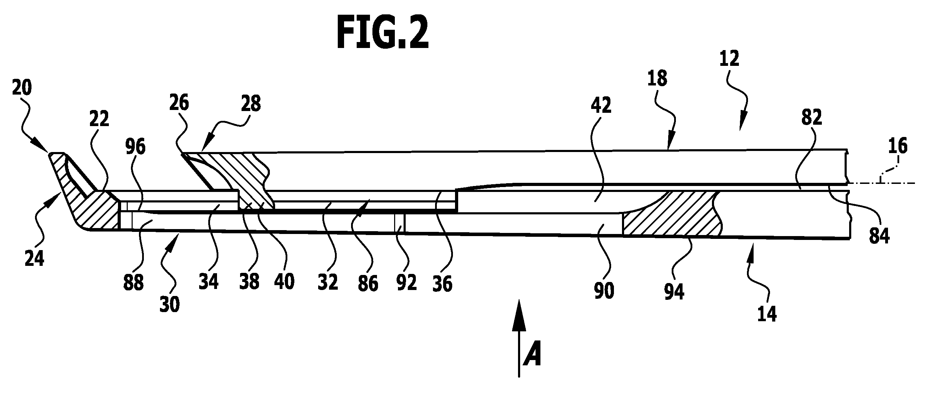

- Figur 2:: eine vergrößerte Teilansicht eines distalen Endbereichs der Knochenstanze aus Figur 1;

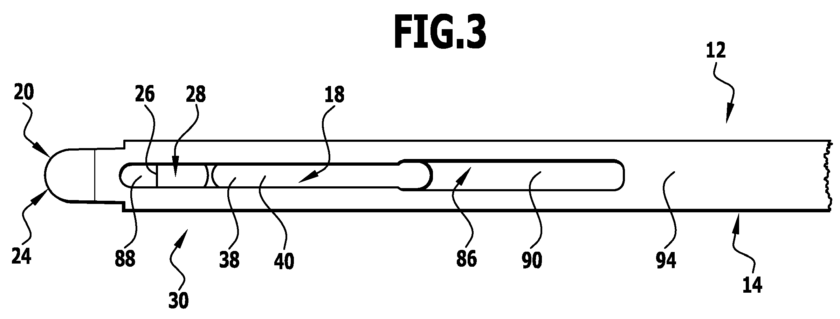

- Figur 3:: eine Ansicht in Richtung des Pfeils A in Figur 2;
- Figur 4:: eine teilweise durchbrochene Seitenansicht eines weiteren Ausführungsbeispiels eines chirurgischen Schiebeschaftinstruments umfassend einen Schiebeschaft und einen lösbar mit diesem verbindbaren Instrumentengriff;
- Figur 5:: eine perspektivische Ansicht des Schiebeschafts aus Figur 4;
- Figur 6:: eine Schnittansicht der Längslinie 6 - 6 in Figur 5;
- Figur 7:: eine teilweise Schnittansicht der längs Linie 7 - 7 in Figur 6; und
- Figur 8:: eine Schnittansicht analog Figur 6 eines weiteren Ausführungsbeispiels eines Schiebeschafts.

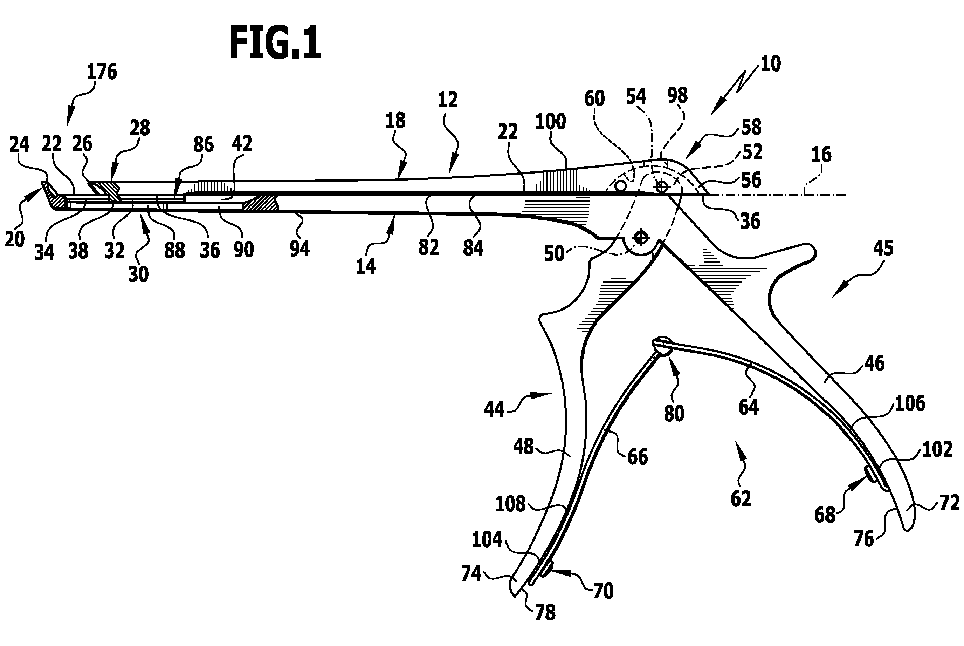

In Figur 1 ist ein insgesamt mit dem Bezugszeichen 10 versehenes erstes Ausführungsbeispiel eines chirurgisches Schiebeschaftinstrument in Form einer Knochenstanze 10 dargestellt, die auch als Osteotom bezeichnet wird. Sie umfasst einen Schiebeschaft 12 mit einem ersten Schaftteil 14 und einem relativ zu diesem parallel zu einer vom Schiebeschaft 12 definierten Längsachse 16 verschiebbar angeordneten zweiten Schaftteil 18.

Der erste Schaftteil 14 ist langgestreckt quaderförmig ausgebildet und weist an seinem distalen Ende ein erstes Werkzeugelement 20 auf, und zwar in Form eines von einer Oberseite 22 abstehenden ambossartigen Gegenlagers 24 für eine an einem distalen Ende des zweiten Schaftteils 18 angeordnete Schneide 26. In einer Ruhestellung, wie sie in Figur 1 dargestellt ist, sind die Schneide 26 und das Gegenlager 24 voneinander beabstandet. Wird der zweite Schaftteil 18 relativ zum ersten Schaftteil 14 in distaler Richtung bewegt, kann die Schneide 26, die ein zweites Werkzeugelement 28 bildet, direkt gegen das Gegenlager 24 drücken und so Knochen- oder Knorpelmaterial in gewünschter Weise bearbeiten. Die Werkzeugelemente 20 und 28 bilden gemeinsam ein Stanzwerkzeug 176 aus.

Eine Führungseinrichtung 30 zum Führen einer Bewegung des zweiten Schaftteils 18 relativ zum ersten Schaftteil 14 umfasst ein erstes Führungselement 32 in Form einer hinterschnittenen oder im Querschnitt T-förmigen Führungsnut 34, welche in Richtung auf den zweiten Schaftteil 18 hin weisend offen ist. Die Führungsnut 34 erstreckt sich etwa auf einem Drittel der Gesamtlänge des ersten Schaftteils 14. Von einer Unterseite 36 des zweiten Schaftteils 18 steht ein zweites Führungselement 38 in Form eines Führungsvorsprungs 40 ab, welcher formschlüssig in die Führungsnut 34 eingreift derart, dass die beiden Schaftteile 14 und 18 nur parallel zur Längsachse 16 relativ zueinander verschiebbar sind. Proximalseitig der Führungsnut 34 schließt sich an diese eine Einführvertiefung 42 an, welche dazu dient, den Führungsvorsprung 40 bei der Montage der Knochenstanze 10 aufzunehmen, bis die Unterseite 36 an die Oberseite 22 in Anlage gebracht und dann der Führungsvorsprung 40 durch Bewegen des zweiten Schaftteils 18 in distaler Richtung mit der Führungsnut 34 in Eingriff gebracht werden kann.

An einem proximalen Ende des Schiebeschafts 12 ist ein insgesamt mit dem Bezugszeichen 44 versehenes Griffteil angeordnet, welches einen unlösbar mit dem Schiebeschaft verbundenen Instrumentengriff 45 bildet und ein erstes Griffelement 46 sowie ein zweites Griffelement 48 umfasst, welche Griffelemente 46 und 48 auch als sogenannte Branchen bezeichnet werden. Das erste Griffelement 46 ist einstückig mit dem ersten Schaftteil 40 ausgebildet und steht seitlich von diesem in etwa unter einem Winkel von 45° bezogen auf die Längsachse 16 ab. Das zweite Griffelement 48 ist um eine Schwenkachse 50, welche in einer Richtung senkrecht zur Längsachse 16 verläuft, am ersten Schaftteil 14 im Übergangsbereich zum ersten Griffelement 46 verschwenkbar gelagert. Ein sich über die Schwenkachse 50 hinaus erstreckendes Kupplungsende 52 des zweiten Griffelements 48 ist um eine parallel zur Schwenkachse 50 verlaufende Schwenkachse 54 verschwenkbar mit einem proximalen Ende 56 des zweiten Schaftteils 18 gekoppelt und definiert einen Schlussbereich 58. Das Ende 56 steht unabhängig von einer Schwenkstellung der Griffelemente 46 und 48 relativ zueinander stets über die Oberseite 22 vor. Die Schwenkachse 54 verläuft ebenfalls stets oberhalb der Oberseite 22.

Damit die Unterseite 36 auch im Bereich des proximalen Ende 56 an der Oberseite 22 des ersten Schaftteils 14 anliegen kann, ist am zweiten Schaftteil 18 eine in Richtung auf den ersten Schaftteil 14 hin offene Schlussbereichsausnehmung 60 vorgesehen, in die das Ende 56 eintaucht. Die Schwenkachse 54 verläuft innerhalb des von der Schlussbereichsausnehmung 60 definierten Bereichs beziehungsweise Abschnitts des zweiten Schaftteils 18.

Die Knochenstanze 10 umfasst ferner eine Rückstelleinrichtung 62 mit zwei Rückstellgliedern 64 und 66 in Form von Blattfedern. Erste freie Enden der Rückstellglieder 64 und 66 sind jeweils mit einer Schraube 68 beziehungsweise 70 mit freien Enden 72 und 74 der ersten und zweiten Griffelemente 46, 48 verschraubt, und zwar auf Innenflächen 76 und 78 derselben. Die nicht an den Griffelementen 46, 48 festgelegten freien Enden der Rückstellglieder 64 und 66 sind mittels eines Kugelgelenks 80 miteinander verschwenkbar verbunden, und zwar derart, dass in einer Grundstellung die Rückstellrichtung 62 die Enden 72 und 74 voneinander weg hält. Das zweite Griffelement 48 kann dann entgegen der Wirkung der Rückstelleinrichtung 62 in Richtung auf das erste Griffelement 46 hin verschwenkt werden, wobei gleichzeitig auf Grund der gelenkigen Verbindung des zweiten Griffelements 48 mit dem zweiten Schaftteil 18 dieser in distaler Richtung bewegt werden kann. Lässt eine Bedienperson den Griffteil 44 der Knochenstanze 10 los, zwingt die Rückstelleinrichtung 62 die Knochenstanze 10 wieder in die Grundstellung zurück, die in Figur 1 dargestellt ist.

Um die Reinigbarkeit der Knochenstanze 10 zu verbessern, sind an dieser gegenüber aus dem Stand der Technik bekannten Knochenstanzen verschiedene Veränderungen vorgenommen.

Zum einen umfasst die Knochenstanze 10 einen Reinigungsspalt 82, welcher durch eine flache Vertiefung 84 an der Unterseite 36 des zweiten Schaltteils 18 ausgebildet ist, und zwar über die gesamte Breite des Schaftteils 18 quer zur Längsachse 16. Die Vertiefung 84 weist eine Tiefe in einem Bereich zwischen 0,3 mm und 0,9 mm auf, so dass ein durch den Reinigungsspalt 82 definierter Abstand zwischen dem ersten Schaftteil 14 und dem zweiten Schaftteil 18 im Bereich des Reinigungsspalts 82 der Tiefe der Vertiefung 84 entspricht. Der Reinigungsspalt 82 erstreckt sich in Richtung der Längsachse 16 ausgehend etwa von der Schlussbereichsausnehmung 60 bis nahe an den Führungsvorsprung 40 heran. Ein Führungsbereich 86 der Knochenstanze 10 wird dann definiert durch einen sich distalseitig an die Vertiefung 84 anschließenden Abschnitt des zweiten Schaftteils 18 in Verbindung mit der Führungsnut 34. Die Breite des Reinigungsspalts 82 beziehungsweise die Tiefe der Vertiefung 84 ist so gewählt, dass eine Kapilarwirkung für eine Reinigungsflüssigkeit vermieden wird. Die der Oberseite 22 gegenüberliegende Vertiefung 84 ist so zum Reinigen mittels einer Reinigungs- oder Spülflüssigkeit frei zugänglich.

Zum anderen ist die Führungsnut 34 vom zweiten Schaftteil 18 weg weisend seitlich geöffnet, und zwar mittels zweier Spülöffnungen 88 und 90, die durch einen Quersteg 92 voneinander getrennt sind. Die in Form von Schlitzen oder Langlöchern ausgebildeten Spülöffnungen 88 und 90 bilden eine Fluidverbindung zwischen einer Unterseite 94 des ersten Schaftteils 14 und einem von der Führungsnut 34 definierten Führungsraum 96 aus. Damit lässt sich auch die Führungsnut 34 mittels einer Reinigungs- und Spülflüssigkeit optimal reinigen. Die proximalseitig des Stegs vorgesehene Spülöffnung 90 steht zudem in Fluidverbindung mit der Einführvertiefung 42, welche mindestens teilweise der Vertiefung 84 gegenüberliegt, und zwar unabhängig von einer Stellung der Schaftteile 14 und 18 relativ zueinander.

Um ferner noch die Reinigbarkeit der Knochenstanze 10 im Schlussbereich 58 zu verbessern, ist eine, zum Beispiel in Form einer Bohrung oder eines Schlitzes ausgebildete, Schlussbereichsöffnung 98 in Form einer die Schlussbereichsausnehmung 60 eröffnenden Durchbrechung vorgesehen, welche eine Fluidverbindung zwischen der Schlussbereichsausnehmung 60 und einer Oberseite 100 des zweiten Schaftteils 18 herstellt. Die Schlussbereichsausnehmung 60 kann so optimiert gereinigt werden, da sie zum einen mit der Vertiefung 84 in Fluidverbindung steht und zum anderen mit der Oberseite 100.

Auch im Bereich des Griffteils 44 wurde die Reinigbarkeit der Knochenstanze 10 im Vergleich zu bekannten Schiebeschaftinstrumenten verbessert. Zwischen die mit den Schrauben 68 und 70 fixierten Enden der Rückstellglieder 64 und 66 und den Griffelementen 46 und 48 ist jeweils ein Abstandselement, beispielsweise in Form einer Unterlegscheibe 102 beziehungsweise 104, angeordnet, welche die Rückstellglieder 62 und 64 von den Griffelementen 46 und 48 beabstandet halten, zumindest in der in der Figur 1 dargestellten Grundstellung.

Zwischen den Rückstellgliedern 64 und 66 sowie den Griffelementen 46 und 48 werden auf diese Weise Spalte 106 und 108 ausgebildet, deren Breite, also ein Abstand zwischen den Rückstellgliedern 64 und 66 einerseits den Griffelementen 46 und 48 andererseits in etwa einer Dicke der Unterlegscheiben 102 und 104 entspricht. Die Dicke der Unterlegscheiben 102 und 104 liegt vorzugsweise in einem Bereich zwischen 0,4 mm und 1 mm.

Sämtliche Teile der Knochenstanze 10 sind vorzugsweise aus einem nichtrostenden Instrumentenstahl hergestellt.

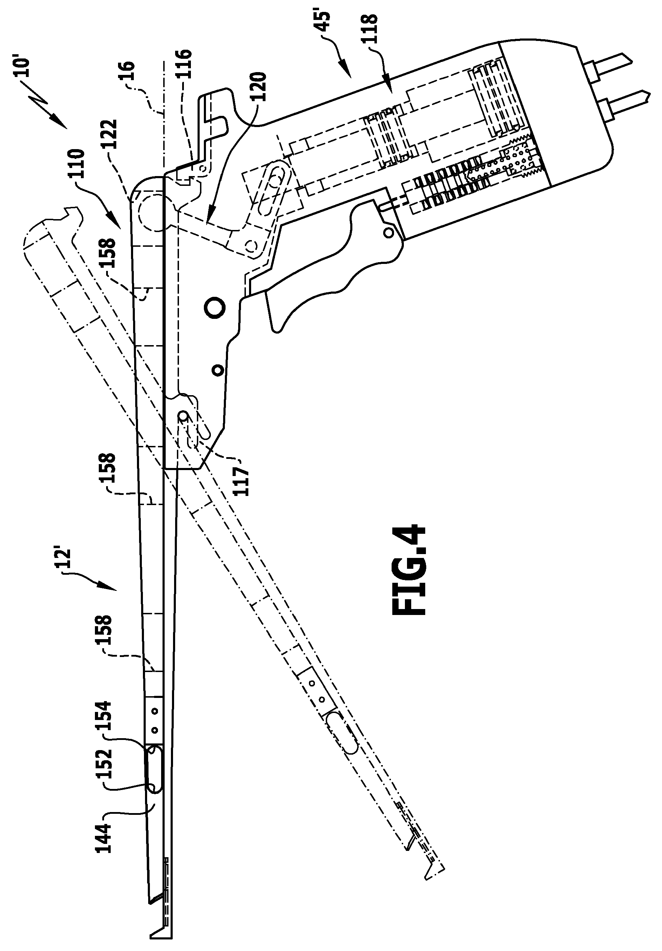

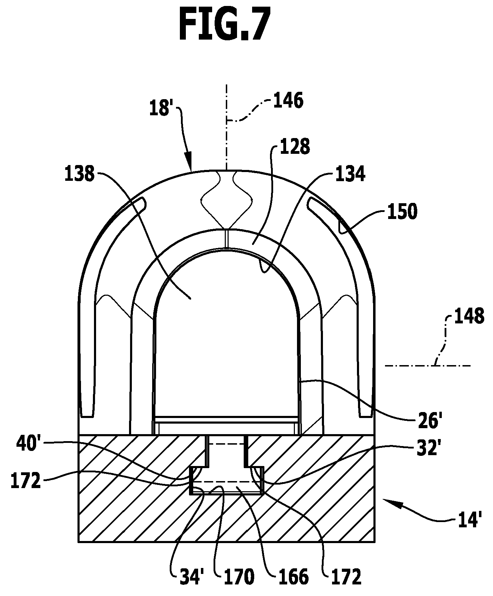

In den Figuren 4 bis 7 ist ein weiteres Ausführungsbeispiel einer insgesamt mit dem Bezugszeichen 10' versehenen Knochenstanze dargestellt, welche einen Schiebeschaft 12' und einen mit diesem lösbar verbindbaren Instrumentengriff 45' umfasst. Der Instrumentengriff 45' und der Schiebeschaft 12' sind mittels einer Kupplungseinrichtung 110 miteinander lösbar verbindbar.

Der Schiebeschaft 12' entspricht in seinem grundsätzlichen Aufbau dem Schiebeschaft 12 und umfasst einen ersten Schaftteil 14' sowie einen zweiten Schaftteil 18' die über eine Führungseinrichtung 30' miteinander gekoppelt sind. An einem proximalen Ende des ersten Schaftteils 14' beziehungsweise etwas beabstandet von diesem sind Kupplungselemente 112 und 114 definierende und von einer Unterseite 94' des ersten Schaftteils 14' abstehende Kupplungsvorsprünge ausgebildet, die mit korrespondierenden, am Instrumentgriff 45' vorgesehenen Kupplungselementen 116 und 117 in Eingriff bringbar sind, um den ersten Schaftteil 14 unbeweglich relativ zum Instrumentengriff 45' an diesem anzuordnen.

Der Instrumentgriff 45' umfasst einen Antrieb 118 in Form eines Pneumatikzylinders, welcher mit einem abgewinkelten und verschwenkbar gelagerten Antriebsglied 120 gekoppelt ist, welches wiederum mit einer Antriebsgliedaufnahme 122, welche in einem proximalen Endbereich des zweiten Schaftteils 18' ausgebildet ist, in Eingriff bringbar ist. Der Antrieb 118 kann so genutzt werden, um den zweiten Schaftteil 18' relativ zum ersten Schaftteil 14' parallel zur Längsachse 16 in distaler Richtung zu bewegen. Ein Beispiel für einen pneumatisch angetriebenen beziehungsweise betätigbaren Instrumentengriff ist in der DE 20 2004 015 643 U1 beschrieben, die mit ihrem gesamten Offenbarungsgehalt in diese Beschreibung mit einbezogen wird.

Der erste Schaftteil 14' ist in Form eines langgestreckten, im Wesentlichen quaderförmigen Körpers 124 ausgebildet und weist an seinem distalen Ende ein erstes Werkzeugelement 20' auf, und zwar in Form eines von einer Oberseite 22' abstehenden ambossartigen Gegenlagers 24' für eine an einem distalen Ende des zweiten Schaftteils 18' angeordnete Schneide 26'. In einer Ruhestellung, wie sie in Figur 4 dargestellt ist, sind die Schneide 26' und das Gegenlager 24' voneinander beabstandet. Wird der zweite Schaftteil 18' relativ zum ersten Schaftteil 14' in distaler Richtung bewegt, kann die Schneide 26', die ein zweites Werkzeugelement 28' bildet, direkt gegen das Gegenlager 24 drücken und so Knochen und Knorpelmaterial in gewünschter Weise bearbeiten. Es sei angemerkt, dass die Schneide 26' eine Schneidebene 126 definiert, welche bezogen zur Längsachse 16 etwas geneigt ist.

Eine Führungseinrichtung 30' zum Führen einer Bewegung des zweiten Schaftteils 18' relativ zum ersten Schaftteil 14' umfasst ein erstes Führungselement 32' in Form einer hinterschnittenen oder im Querschnitt T-förmigen Führungsnut 34', welche in Richtung auf den zweiten Schaftteil 18' hin weisend offen ist. Die Führungsnut 34' erstreckt sich etwa auf einem Fünftel der Gesamtlänge des ersten Schaftteils 14'. Von einer Unterseite 36' eines einen distalen Endabschnitt des ersten Schaftteils 18' bildenden Werkzeugteils 128 steht ein zweites Führungselement 38' in Form eines Führungsvorsprungs 40' ab, welcher im Wesentlichen formschlüssig in die Führungsnut 34' eingreift derart, dass die beiden Schaftteile 14' und 18' nur parallel zur Längsachse 16 relativ zueinander verschiebbar sind.

Proximalseitig der Führungsnut 34' schließt sich an diese eine Einführvertiefung 42' an, welche dazu dient, den Führungsvorsprung 40' bei der Montage des Schiebeschafts 12' aufzunehmen, bis die Unterseite 36' an die Oberseite 22' des ersten Schaftteils 18' in Anlage gebracht und dann der Führungsvorsprung 40' durch Bewegen des Schaftteils 18' in distaler Richtung mit der Führungsnut 34' in Eingriff gebracht werden kann.

Der zweite Schaftteil 18' ist zweiteilig ausgebildet und umfasst das Werkzeugteil 128 sowie einen sich an diesen proximalseitig anschließenden Schiebeteil 130, an welchem die Antriebsgliedaufnahme 122 ausgebildet ist. Das Werkzeugteil 128 ist hülsenartig geformt und umfasst die Schneide 26' an seinem distalen Ende, die durch Anschleifen einer Außenseite 132 des Werkzeugteils 128 im Bereich von dessen distalem Ende ausgebildet wird. Die Schneide 26' begrenzt eine Aufnahmeöffnung 134 eines sich in proximaler Richtung erstreckenden Innenraums 136 des Werkzeugteils 128, welcher eine Schneidgutaufnahme 138 bildet.

Eine innere Querschnittsfläche 140 der Schneidgutaufnahme 138 nimmt ausgehend von der Aufnahmeöffnung 134 in proximaler Richtung kontinuierlich zu, das heißt sie wird größer. Die kleinste Querschnittsfläche definiert somit die Aufnahmeöffnung 134. Die größte Querschnittsfläche 140 weist die Schneidgutaufnahme 138 an ihrem proximalen Ende 142 auf. Von diesem ausgehend in distaler Richtung erstrecken sich in einer Wand 144 des zweiten Schaftteils 18' symmetrisch zu einer Mittelebene 146, welche gleichzeitig eine Symmetrieebene des Schiebeschafts 12' definiert, in einer parallel zu einer senkrecht zur Mittelebene 146 verlaufenden Entleerungsrichtung 148 geöffnete Entleerungsöffnungen 150. Eine Höhe der Entleerungsöffnungen 150 in einer Richtung sowohl senkrecht zur Entleerungsrichtung 148 als auch zur Längsachse 16 entspricht einer Höhe der Schneidgutaufnahme 138. Die Entleerungsöffnungen 150 sind etwa drei Mal so lang wie breit, wobei distale und proximale Enden 152 und 154 ausgerundet sind.

Proximalseitig schließt sich an das proximale Ende 142 ein Verbindungsvorsprung 156 an, an welchem das Schiebeteil 130 durch Anspritzen angeformt ist. Das Werkzeugteil 128 ist vorzugsweise aus einem Instrumentenstahl hergestellt, das Schiebeteil 130 aus einem spritzfähigen Kunststoff. Das Schiebeteil 130 ist somit aus einem Schiebeteilmaterial hergestellt, das Werkzeugteil 128 aus einem Werkzeugteilmaterial, die im vorliegenden Fall unterschiedlich gewählt sind, nämlich einerseits ein Kunststoff und andererseits ein Metall. Alternativ kann das Schiebeteil 130 auch separat gespritzt und anschließend mit dem Werkzeugteil 128 durch Kleben oder Schweißen im Bereich des Verbindungsvorsprungs 156 mit dem Werkzeugteil 128 verbunden werden. Selbstverständlich kann das Schiebeteil 130 auch aus einem Metall hergestellt sein, so dass es mit dem Werkzeugteil 128 durch Kleben, Löten oder Schweißen verbunden werden kann.

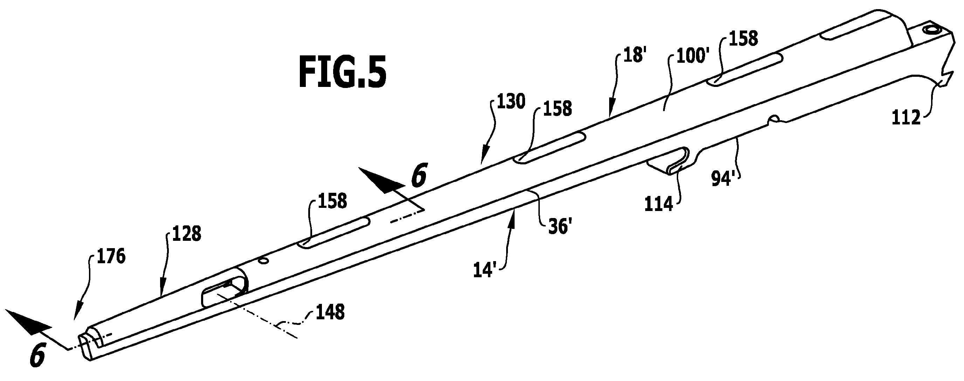

Proximalseitig des Verbindungsvorsprungs 156 sind am Schiebeteil 130 langlochartige Spüldurchbrechungen 158 ausgebildet, bei dem in Figur 5 dargestellten Ausführungsbeispiel des Schiebeschafts 12' insgesamt drei Spüldurchbrechungen 158, die sich von der Oberseite 100' des zweiten Schaftteils 18' bis zu dessen Unterseite 36' erstrecken.

Zur besseren Führung einer Bewegung des zweiten Schaftteils 18' relativ zum ersten Schaftteil 14' können am ersten Schaftteil 14' in Richtung auf das zweite Schaftteil 18' weisend abstehende, im Querschnitt senkrecht zur Längsachse 16 T-förmige Vorsprünge ausgebildet sein, die sich parallel zur Längsachse 16 über etwa ein Drittel der Länge der Spüldurchbrechungen erstrecken. Zur Ausbildung von in den Figuren 4 und 5 nicht dargestellten Führungseinrichtungen können optional an sich parallel zur Längsachse 16 erstreckenden inneren Seitenwänden abstehende und aufeinander zu weisende stegförmige Führungsvorsprünge angeordnet oder ausgebildet sein, die zwischen sich einen sich parallel zur Längsachse 16 erstreckenden Führungsschlitz für jeweils einen der T-förmigen Vorsprünge definieren. Mit anderen Worten definieren die Führungsvorsprünge T-förmige Führungsnuten für die T-förmigen Vorsprünge. Die Führungsvorsprünge erstrecken sich über etwas weniger als zwei Drittel der Länge der Spüldurchbrechung 158 parallel zur Längsachse, und zwar ausgehend vom proximalen Ende der jeweiligen Spüldurchbrechung 158. Auf diese Weise können die T-förmigen Vorsprünge von unten im Bereich distalet Enden der Spüldurchbrechungen 158 gleichzeitig eingeführt und durch eine Bewegung des zweiten Schaftteils 18' relativ zum ersten Schaftteil 14' in distaler Richtung in den zwischen den Führungsvorsprüngen definierten Führungsschlitz eingeführt werden. Auf diese Weise werden der erste Schaftteil 14' und der zweite Schaftteil 18' auch im Bereich des Schiebeteils geführt aneinander gehalten.

Durch die besondere Anordnung und Ausgestaltung der Spüldurchbrechungen 158 können die im Bereich derselben durch die T-förmigen Vorsprünge am ersten Schaftteil 14' und die an den inneren Seitenwänden der Spüldurchbrechungen 158 vorgesehenen Führungsvorsprünge optional vorgesehenen Führungseinrichtungen durchgehend offen ausgebildet werden, wodurch eine Reinigung der Knochenstanze 10 erleichtert wird.

Der zweite Schaftteil 18', welcher das Schiebeteil 130 sowie das Werkzeugteil 128 umfasst, kann, wie bereits beschrieben, insbesondere durch Anspritzen des Schiebeteils 130 an das Werkzeugteil 128 hergestellt werden. Denkbare Spritzverfahren sind zum Beispiel Kunststoffspritzgießen, Metallspritzgießen ("metal injection molding" oder weitere sogenannte "MIM"-Technologien) oder Keramikspritzgießen mit anschließendem Sintern ("ceramic injection molding" oder weitere sogenannte "CIM"-Technologien) hergestellt werden. Dabei wird jeweils ein Spritzgusswerkzeug benötigt. Die Spüldurchbrechungen 158 insbesondere im Bereich der durch die T-förmigen Vorsprünge am ersten Schaftteil 14' und die an den inneren Seitenwänden der Spüldurchbrechungen 158 vorgesehenen Führungsvorsprünge optional vorgesehenen Führungseinrichtungen vorzusehen hat den Vorteil, dass das Spritzgusswerkzeug für den Schiebeteil so relativ einfach, nämlich insbesondere ohne Werkzeugschieber, aufgebaut werden kann.

Die Schneidgutaufnahme 138 könnte optional in Richtung auf den ersten Schaftteil 14' geöffnet und von diesem verschlossen sein. Ferner ist im vorliegenden Fall eine Projektionsfläche einer Projektion der Aufnahmeöffnungsquerschnittsfläche auf eine Ebene senkrecht zur Längsrichtung 16 kleiner als eine beliebige Querschnittsfläche 140 eines beliebigen Querschnitts der Schneidgutaufnahme 138 parallel zur Projektionsfläche. Wie bereits dargelegt, nimmt eine innere Querschnittsfläche 140 der Schneidgutaufnahme 138 ausgehend von der Aufnahmeöffnung 134 in proximaler Richtung zu, bei dem in den Figuren dargestellten Ausführungsbeispiel sogar monoton zu. Im vorliegenden Fall erweitert sich die Schneidgutaufnahme 138 ausgehend von der Aufnahmeöffnung 134 in proximaler Richtung konisch. Die Entleerungsöffnungen 150 sind somit in einem Bereich der Schneidgutaufnahme 138 ausgebildet, welche die größte innere Querschnittsfläche 140 aufweist.

Die Entleerungsöffnungen 150 weisen zudem seitwärts, mit anderen Worten, sie sind in seitlicher Richtung hin weisend geöffnet. Sie gestatten auf einfache Weise das Entleeren der Schneidgutaufnahme 138, wenn sich diese in Form von mehreren, nacheinander durchgeführten Stanzungen mit Schneidgut gefüllt hat. Insbesondere kann ein distales Ende des Schiebeschafts 12' in einem Operationsgebiet verbleiben, wenn beispielsweise ein Assistent eines Operateurs die Schneidgutaufnahme 138 teilweise dadurch leert, dass er mit einem Hilfsinstrument, beispielsweise einem kleinen Stößel, in die Schneidgutaufnahme 138 eingebrachtes Schneidgut seitlich durch Einführen des Stößels durch eine Entleerungsöffnung 150 und Herausdrücken des Schneidguts durch die andere Entleerungsöffnung 150 aus der Schneidgutaufnahme 138 herausdrückt. Die Knochenstanze 10' wird so schnell und auf einfache Weise vorbereitet für weitere Stanzvorgänge, die der Operateur ausführen kann.

Am Schiebeschaft 12' befindet sich das eigentliche Verschleißteil des Schiebeschafts 12', nämlich die Schneide 26'. Durch Ausstanzen von hartem Knochenmaterial kann diese stumpf werden, so dass der gesamte Schiebeschaft 12' praktisch unbrauchbar wird. Grundsätzlich besteht die Möglichkeit, die Schneide 26' nachzuschärfen, beispielsweise durch Nachschleifen der Außenseiten 132.

Aufgrund der zweiteiligen Ausgestaltung des zweiten Schaftteils 18' kann dieser jedoch auch als Einwegartikel zur Verfügung gestellt werden, so dass der praktisch keinem Verschleiß unterworfene erste Schaftteil 14' weiter verwendet, der zweite Schaftteil 18' jedoch durch einen neuen zweiten Schaftteil 18' mit einer noch scharfen Schneide 26' ersetzt werden kann. Die zweiteilige Ausbildung des zweiten Schaftteils 18' gestattet es so, nur das Werkzeugteil aus einem zur Ausbildung einer Schneide 26' geeigneten Material vorzusehen, den übrigen Teil des zweiten Schaftteils 18' aus einem kostengünstigeren und leicht bearbeitbaren Material, beispielsweise einem Kunststoff.

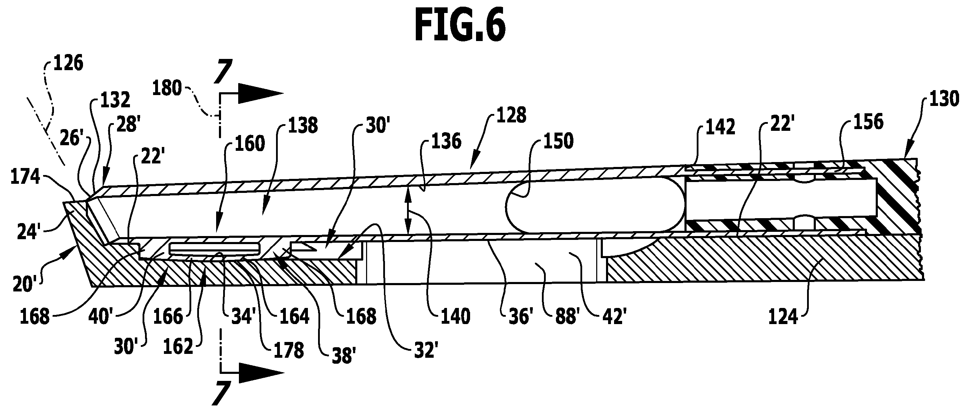

Um erste Schaftteile 14' und zweite Schaftteile 18' praktisch beliebig miteinander kombinieren zu können, ist es erforderlich, im Bereich der Führungseinrichtung 30' ausreichende Fertigungstoleranzen vorzusehen beziehungsweise zuzulassen, um die ersten und zweiten Führungselemente 32' und 38' wahlweise miteinander in Eingriff bringen zu können. Um trotzdem zu gewährleisten, dass mit dem Schiebeschaft 12' Gewebe und Knochen in gewohnt präziser Weise bearbeitet werden können, ist eine Toleranzausgleichseinrichtung 160 zum Ausgleichen von Fertigungstoleranzen der zusammenwirkenden Führungselemente 32' und 38' vorgesehen. Sie sind insbesondere derart angeordnet und ausgebildet, dass Fertigungstoleranzen in der Arbeitsstellung, in welcher die ersten und zweiten Werkzeugelemente 20' und 28' zum Bearbeiten von Gewebe oder Knochen zusammenwirken, also insbesondere aneinander anliegen, wie in Figur 6 dargestellt, ausgeglichen werden.

Die Toleranzausgleichseinrichtung 160 umfasst eine Andrückeinrichtung 162 zum Vorspannen des zweiten Führungselements 38' gegen das erste Führungselement 32'. Sie ist bei dem in den Figuren dargestellten Ausführungsbeispiel am zweiten Führungselement 38' angeordnet beziehungsweise ausgebildet, könnte jedoch auch am ersten Führungselement 32' angeordnet oder ausgebildet sein. Die Andrückeinrichtung 162 umfasst ein vorspannendes Element 164 zum Vorspannen des zweiten Führungselements 38' gegen das erste Führungselement 32'. Das vorspannende Element 164 wiederum ist in Form eines Federelements 166 ausgebildet, und zwar in Form einer Blattfeder, welche sich zwischen zwei von der Unterseite 36' des Werkzeugteils 128 abstehenden Vorsprüngen 168 erstreckt, die beide einen Teil der Führungseinrichtung 30' beziehungsweise des Führungsvorsprungs 40' bilden. Das Federelement 160 ist von der Unterseite 36' weg weisend schwach konvex gekrümmt und liegt an einem in Richtung auf das zweite Schaftteil 18 hin weisenden Boden 170 der Führungsnut 34' an. Der Führungsvorsprung 40' wird so gegen in Richtung auf den Boden 170 hin weisende innere Begrenzungsflächen 172 der Führungsnut 34' gedrückt und gleicht Fertigungstoleranzen im Bereich der Führungsnut 34' beziehungsweise des Führungsvorsprungs 40' aus. Durch die spezielle Ausbildung der Toleranzausgleichseinrichtung 160 kann in der Arbeitsstellung die Unterseite 36' im Bereich des Werkzeugteils 128 etwas vom ersten Schaftteil 14' beabstandet sein.

Das Gegenlager 24', auch als Ambossglied bezeichnet, ist so groß ausgebildet, dass die Schneide 26' des zweiten Schaftteils 18' nicht über eine Oberkante 174 des Gegenlagers 24' geschoben werden kann, um eine Schneidwirkung der ein Stanzwerkzeug 176 ausbildenden Werkzeugelemente 20' und 28' in jedem Fall sicherzustellen. Ein Toleranzausgleich der aufgrund des Federelements 166 zumindest teilweise elastischen Führungseinrichtung 30' ist also höchstens so groß wie ein Abstand der Schneide 26' bis zur Oberkante 174.

Um insbesondere den ersten, wiederverwendbaren Schaftteil 14' leicht reinigen zu können, ist proximalseitig der Führungsnut 34' eine Spülöffnung 88' vorgesehen analog der Spülöffnung 88 bei der Knochenstanze 10. Sie steht insbesondere mit der Führungsnut 34' in Fluidverbindung.

Das vorspannende Element 164 und das zweite Führungselement 38' sind bei dem in den Figuren dargestellten Ausführungsbeispiel einstückig ausgebildet. Das vorspannende Element 164 ist ferner entgegen einer von ihm ausübbaren Kraft von einer Grundstellung, in welcher ein Abstand zwischen einer vom zweiten Schaftteil 18' weg weisenden Andrückfläche 178 und der Unterseite 36' des zweiten Schaftteils 18' maximal ist, in eine Andrückstellung bringbar, wie sie beispielsweise in Figur 6 dargestellt ist, in welcher ein Abstand zwischen der vom zweiten Schaftteil 18' weg weisenden Andrückfläche 178 und der Unterseite 36' des zweiten Schaftteils 18' minimal ist.

Das vorspannende Element 164 und insgesamt die Toleranzausgleichseinrichtung 160 sind spiegelsymmetrisch zu einer senkrecht zur Längsachse 16 verlaufenden Spiegelebene 180 ausgebildet. Ebenso sind proximale und distale Enden des vorspannenden Elements 164 am zweiten Führungselement 38' gehalten.

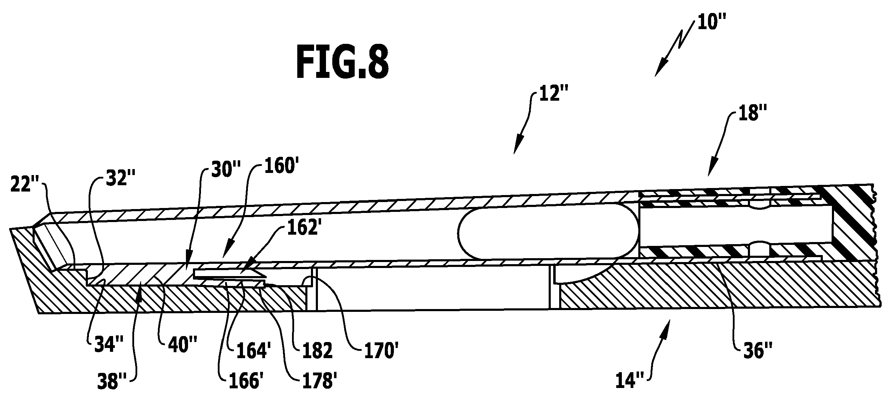

Ein alternatives Ausführungsbeispiel eines Schiebeschafts einer Knochenstanze 10" ist in Figur 8 teilweise dargestellt und insgesamt mit dem Bezugszeichen 12" gekennzeichnet. Der Schiebeschaft 12" unterscheidet sich lediglich in der Ausgestaltung der Führungseinrichtung 30" sowie der Toleranzausgleichseinrichtung 160 vom Schiebeschaft 12', so dass dieselben Bezugszeichen für einander entsprechende Teile beziehungsweise mit zwei Anstrichen (""") versehene identische Bezugsziffern zur Kennzeichnung verwendet werden.

Vom Führungsvorsprung 40" steht in proximaler Richtung und etwas in Richtung auf den ersten Schaftteil 14" hin weisend ein vorspannendes Element 164' in Form eines Federelements 166' ab, welches blattfederartig ausgebildet ist. Es stützt sich am Boden 170' der Führungsnut 34" mit einer Andrückfläche 178' ab. Die Andrückeinrichtung 162' der Toleranzausgleichseinrichtung 160' entfaltet ihre Wirkung erst dann, wenn das vorspannende Element 164 an seinem proximalen Ende 182 mit der Führungsnut 34" in Eingriff kommt, also erst kurz vor Erreichen der in Figur 8 dargestellten Arbeitsstellung sowie in der Arbeitsstellung und drückt dann die Unterseite 36" etwas von der Oberseite 22" weg.

Der Schiebeschaft 12" ist analog dem Schiebeschaft 12' mit dem Instrumentengriff 45' lösbar verbindbar.

## Patentansprüche

1. Schiebeschaft (12'; 12") für ein chirurgisches Schiebeschaftinstrument (10'; 10"), welcher eine Längsrichtung (16) definiert, mit einem ersten Schaftteil (14'; 14") und einem zweiten Schaftteil (18'; 18"), die relativ zueinander verschiebbar angeordnet sind, welcher erste Schaftteil (14'; 14") ein erstes Werkzeugelement (20'; 20") trägt und welcher zweite Schaftteil (18'; 18") ein zweites Werkzeugelement (28'; 28") trägt, wobei der zweite Schaftteil (18'; 18") eine Schneidgutaufnahme (138) aufweist mit einer Aufnahmeöffnung (134), welche vom zweiten Werkzeugelement (28'; 28") begrenzt ist und sich ausgehend von diesem in proximaler Richtung erstreckt, wobei der zweite Schaftteil (18'; 18") mindestens zweiteilig ausgebildet ist und ein Werkzeugteil (128), welches das zweite Werkzeugelement (28'; 28") umfasst, und ein mit dem Werkzeugteil (128) verbundenes Schiebeteil (130) umfasst, **dadurch gekennzeichnet, dass** das Werkzeugteil (128) und das Schiebeteil (130) unlösbar miteinander verbunden sind und dass das Werkzeugteil (128) und das Schiebeteil (130) durch Schweißen, Löten oder Kleben miteinander verbunden sind oder dass das Schiebeteil (130) durch Anspritzen an das Werkzeugteil (128) hergestellt ist.

2. Schiebeschaft nach Anspruch 1, **dadurch gekennzeichnet, dass** die Schneidgutaufnahme (138) mindestens eine Entleerungsöffnung (150) aufweist, welche proximalseitig der Aufnahmeöffnung (134) ausgebildet ist, und dass die mindestens eine Entleerungsöffnung (150) in einer seitlichen Entleerungsrichtung (148) weisend geöffnet ist, welche Entleerungsrichtung (148) senkrecht oder im Wesentlichen senkrecht zur Längsrichtung (16) und parallel oder im Wesentlichen parallel zu einer von einer Unterseite (36'; 36") des zweiten Schaftteils (18'; 18") definierten Anlageebene orientiert ist.

3. Schiebeschaft nach einem der voranstehenden Ansprüche, **dadurch gekennzeichnet, dass** die Schneidgutaufnahme (138) in Richtung auf den ersten Schaftteil (14'; 14") hin weisend offen ist und dass der erste Schaftteil (14'; 14") die Schneidgutaufnahme (138) vollständig verschließt.

4. Schiebeschaft nach einem der voranstehenden Ansprüche, **dadurch gekennzeichnet, dass** eine Aufnahmeöffnungsquerschnittsfläche der Aufnahmeöffnung (134) kleiner ist als eine Querschnittsfläche (140) eines beliebigen Querschnitts der Schneidgutaufnahme (138) parallel zur Aufnahmeöffnungsquerschnittsfläche.

5. Schiebeschaft nach einem der voranstehenden Ansprüche, **dadurch gekennzeichnet, dass** eine innere Querschnittsfläche (140) der Schneidgutaufnahme (138) ausgehend von der Aufnahmeöffnung (134) in proximaler Richtung zunimmt.

6. Schiebeschaft nach einem der voranstehenden Ansprüche, **dadurch gekennzeichnet, dass** die mindestens eine Entleerungsöffnung (150) in einem Bereich der Schneidgutaufnahme (138) ausgebildet ist, welche die größte oder im Wesentlichen die größte Innenquerschnittsfläche (140) aufweist.

7. Schiebeschaft nach einem der voranstehenden Ansprüche, **dadurch gekennzeichnet, dass** sich die mindestens eine Entleerungsöffnung (150) bis zu einem proximalen Ende (142) oder im Wesentlichen bis zu einem proximalen Ende (142) der Schneidgutaufnahme (138) erstreckt.

8. Schiebeschaft nach einem der voranstehenden Ansprüche, **dadurch gekennzeichnet, dass** eine Länge der mindestens einen Entleerungsöffnung (150) parallel zur Längsrichtung (16) mindestens doppelt so groß ist wie eine Breite der Entleerungsöffnung (150) in einer Richtung senkrecht zur Längsrichtung (16) und zur Entleerungsrichtung (148).

9. Schiebeschaft nach einem der voranstehenden Ansprüche, **dadurch gekennzeichnet, dass** in distaler und/oder proximaler Richtung weisende Begrenzungen der mindestens einen Entleerungsöffnung (150) abgerundet sind.

10. Schiebeschaft nach einem der voranstehenden Ansprüche, **dadurch gekennzeichnet, dass** das Schiebeteil (130) aus einem Schiebeteilmaterial und dass das Werkzeugteil (128) aus einem Werkzeugteilmaterial hergestellt ist.

11. Schiebeschaft nach einem der voranstehenden Ansprüche, **dadurch gekennzeichnet, dass** das Werkzeugteil (128) mindestens einen mindestens teilweise umspritzbaren, in proximaler Richtung abstehenden Verbindungsvorsprung (156) aufweist.

12. Schiebeschaft nach einem der voranstehenden Ansprüche, **dadurch gekennzeichnet, dass** das Werkzeugteil (128) proximalseitig offen ist und dass das Schiebeteil (130) das Werkzeugteil (128) proximalseitig verschließt.

13. Schiebeschaft nach einem der voranstehenden Ansprüche, **dadurch gekennzeichnet, dass** das erste Werkzeugelement (20'; 20") und das zweite Werkzeugelement (28'; 28") zusammen ein Stanzwerkzeug (176) ausbilden und dass eine Arbeitsstellung, in welcher das erste und das zweite Werkzeugelement (20', 28'; 20", 28") aneinander anliegen und zusammenwirken, eine Stanzstellung definiert.

14. Chirurgisches Schiebeschaftinstrument (10'; 10") umfassend einen Instrumentengriff (45') und einen mittels des Instrumentengriffs (45') betätigbaren Schiebeschaft (12'; 12"), **gekennzeichnet durch** einen Schiebeschaft (12'; 12") nach einem der voranstehenden Ansprüche.

15. Schiebeschaftinstrument nach Anspruch 14, **dadurch gekennzeichnet, dass** der Instrumentengriff (45') und der Schiebeschaft (12'; 12") miteinander lösbar verbindbar sind.

## Claims

1. Sliding shaft (12'; 12") for a surgical sliding shaft instrument (10'; 10") which defines a longitudinal direction (16) and comprises a first shaft part (14'; 14") and a second shaft part (18'; 18") which are arranged such as to be displaceable relative to each other, wherein said first shaft part (14'; 14") carries a first tool element (20'; 20") and said second shaft part (18'; 18") carries a second tool element (28'; 28"), wherein the second shaft part (18'; 18") comprises a cut material collector (138) having an inlet opening (134) which is bounded by the second tool element (28'; 28") and extends therefrom in the proximal direction, wherein the second shaft part (18'; 18") is formed in at least two parts and comprises a tool part (128) which comprises the second tool element (28'; 28") and a sliding part (130) which is connected to the tool part (128), **characterized in that** the tool part (128) and the sliding part (130) are connected inseparably to one another and **in that** the tool part (128) and the sliding part (130) are connected together by welding, soldering or adhesion and **in that** the sliding part (130) is manufactured by moulding it onto the tool part (128).

2. Sliding shaft according to claim 1, **characterized in that** the cut material collector (138) comprises at least one emptying opening (150) which is formed on the proximal side of the inlet opening (134), and **in that** the at least one emptying opening (150) is open in a lateral emptying direction (148), which emptying direction (148) is oriented perpendicularly or substantially perpendicularly to the longitudinal direction (16) and parallel or substantially parallel to a contact plane defined by a lower surface (36'; 36") of the second shaft part (18'; 18").

3. Sliding shaft according to any one of the preceding claims, **characterized in that** the cut material collector (138) is open in the direction of the first shaft part (14'; 14") and **in that** the first shaft part (14'; 14") fully closes the cut material collector (138).

4. Sliding shaft according to any one of the preceding claims, **characterized in that** an inlet opening cross-sectional area of the inlet opening (134) is smaller than a cross-sectional area (140) of any cross section of the cut material collector (138) parallel to the inlet opening cross-sectional area.

5. Sliding shaft according to any one of the preceding claims, **characterized in that** an internal cross-sectional area (140) of the cut material collector (138) increases from the inlet opening (134) in the proximal direction.

6. Sliding shaft according to any one of the preceding claims, **characterized in that** the at least one emptying opening (150) is formed in a region of the cut material collector (138) that has the largest or substantially the largest internal cross-sectional area (140).

7. Sliding shaft according to any one of the preceding claims, **characterized in that** the at least one emptying opening (150) extends up to a proximal end (142) or substantially up to a proximal end (142) of the cut material collector (138).

8. Sliding shaft according to any one of the preceding claims, **characterized in that** a length of the at least one emptying opening (150) parallel to the longitudinal direction (16) is at least twice as large as a width of the emptying opening (150) in a direction perpendicular to the longitudinal direction (16) and to the emptying direction (148).

9. Sliding shaft according to any one of the preceding claims, **characterized in that** the boundaries of the at least one emptying opening (150) in the distal and/or proximal direction are rounded.

10. Sliding shaft according to any one of the preceding claims, **characterized in that** the sliding part (130) is made of a sliding part material and **in that** the tool part (128) is made of a tool part material.

11. Sliding shaft according to any one of the preceding claims, **characterized in that** the tool part (128) has at least one connecting projection (156) which protrudes in the proximal direction and is at least partially mouldable.

12. Sliding shaft according to any one of the preceding claims, **characterized in that** the tool part (128) is open at the proximal side and **in that** the sliding part (130) closes the tool part (128) at the proximal side.

13. Sliding shaft according to any one of the preceding claims, **characterized in that** the first tool element (20'; 20") and the second tool element (28'; 28") together form a punching tool (176) and **in that** a working position, in which the first and the second tool element (20', 28'; 20", 28") about each other and cooperate, defines a punching position.

14. Surgical sliding shaft instrument (10'; 10") comprising an instrument handle (45') and a sliding shaft (12'; 12") that is operable by means of the instrument handle (45'), **characterized by** a sliding shaft (12'; 12") according to any one of the preceding claims.

15. Sliding shaft instrument according to claim 14, **characterized in that** the instrument handle (45') and the sliding shaft (12'; 12") are connectable to one another in releasable manner.

## Revendications

1. Corps allongé coulissant en forme de tige (12'; 12'') pour un instrument chirurgical à corps allongé coulissant (10'; 10''), qui définit une direction longitudinale (16), et comprenant une première partie de corps allongé (14'; 14") et une deuxième partie de corps allongé (18'; 18"), qui sont agencées de manière à pouvoir coulisser l'une par rapport à l'autre, ladite première partie de corps allongé (14'; 14") portant un premier élément d'outil (20'; 20'') et ladite deuxième partie de corps allongé (18'; 18'') portant un deuxième élément d'outil (28'; 28"),
dans lequel la deuxième partie de corps allongé (18'; 18'') présente un logement d'accueil de matériau coupé (138) avec une ouverture d'accueil (134), qui est délimitée par le deuxième élément d'outil (28'; 28") et s'étend à partir de celui-ci en direction proximale, et dans lequel la deuxième partie de corps allongé (18'; 18'') est réalisée au moins en deux parties et comprend une partie d'outil (128) englobant le deuxième élément d'outil (28'; 28"), et une partie de coulissement (130) reliée à la partie d'outil (128),
**caractérisé en ce que** la partie d'outil (128) et la partie de coulissement (130) sont reliées de manière non amovible, et **en ce que** la partie d'outil (128) et la partie de coulissement (130) sont reliées par soudage, brasage ou collage, ou **en ce que** la partie de coulissement (130) est réalisée en étant moulée par injection sur la partie d'outil (128).

2. Corps allongé coulissant en forme de tige selon la revendication 1, **caractérisé en ce que** le logement d'accueil de matériau coupé (138) présente au moins une ouverture de vidage (150), qui est réalisée du côté proximal de l'ouverture d'accueil (134), et **en ce que** ladite au moins une ouverture de vidage (150) est ouverte dans une direction de vidage latérale (148), cette direction de vidage (148) étant orientée perpendiculairement ou sensiblement de manière perpendiculaire à la direction longitudinale (16), et parallèlement ou sensiblement de manière parallèle à un plan d'appui défini par un côté inférieur (36'; 36") de la deuxième partie de corps allongé (18'; 18").

3. Corps allongé coulissant en forme de tige selon l'une des revendications précédentes, **caractérisé en ce que** le logement d'accueil de matériau coupé (138) est ouvert dans la direction orientée vers la première partie de corps allongé (14'; 14"), et **en ce que** la première partie de corps allongé (14'; 14") ferme complètement le logement d'accueil de matériau coupé (138).

4. Corps allongé coulissant en forme de tige selon l'une des revendications précédentes, **caractérisé en ce qu'**une surface de section transversale d'ouverture d'accueil de l'ouverture d'accueil (134) est plus petite qu'une surface de section transversale (140) d'une section transversale quelconque du logement d'accueil de matériau coupé (138), parallèlement à la surface de section transversale d'ouverture d'accueil.

5. Corps allongé coulissant en forme de tige selon l'une des revendications précédentes, **caractérisé en ce qu'**une surface de section transversale intérieure (140) du logement d'accueil de matériau coupé (138) augmente à partir de l'ouverture d'accueil (134) en direction proximale.

6. Corps allongé coulissant en forme de tige selon l'une des revendications précédentes, **caractérisé en ce que** ladite au moins une ouverture de vidage (150) est formée dans une zone du logement d'accueil de matériau coupé (138), qui présente la plus grande ou sensiblement la plus grande surface de section transversale intérieure (140).

7. Corps allongé coulissant en forme de tige selon l'une des revendications précédentes, **caractérisé en ce que** ladite au moins une ouverture de vidage (150) s'étend jusqu'à l'extrémité proximale (142) ou sensiblement jusqu'à une extrémité proximale (142) du logement d'accueil de matériau coupé (138).

8. Corps allongé coulissant en forme de tige selon l'une des revendications précédentes, **caractérisé en ce qu'**une longueur de ladite au moins une ouverture de vidage (150) parallèlement à la direction longitudinale (16), est au moins égale au double d'une largeur de l'ouverture de vidage (150) dans une direction perpendiculaire à la direction longitudinale (16) et à la direction de vidage (148).

9. Corps allongé coulissant en forme de tige selon l'une des revendications précédentes, **caractérisé en ce que** des délimitations de ladite au moins une ouverture de vidage (150), qui sont orientées en direction distale et/ou proximale, sont arrondies.

10. Corps allongé coulissant en forme de tige selon l'une des revendications précédentes, **caractérisé en ce que** la partie de coulissement (130) est fabriquée en un matériau de partie de coulissement, et la partie d'outil (128) en un matériau de partie d'outil.

11. Corps allongé coulissant en forme de tige selon l'une des revendications précédentes, **caractérisé en ce que** la partie d'outil (128) comporte au moins une proéminence de liaison (156) en saillie dans la direction proximale et pouvant être noyée au moins partiellement par moulage par injection.

12. Corps allongé coulissant en forme de tige selon l'une des revendications précédentes, **caractérisé en ce que** la partie d'outil (128) est ouverte du côté proximal, et **en ce que** la partie de coulissement (130) ferme la partie d'outil (128) du côté proximal.

13. Corps allongé coulissant en forme de tige selon l'une des revendications précédentes, **caractérisé en ce que** le premier élément d'outil (20'; 20'') et le deuxième élément d'outil (28'; 28") forment ensemble un outil de coupe par poinçonnage (176), et **en ce qu'**une position de travail, dans laquelle le premier et le deuxième élément d'outil (20', 28'; 20", 28'') s'appuient et interagissent mutuellement, définit une position de coupe par poinçonnage.

14. Instrument chirurgical à corps allongé coulissant (10'; 10'') comprenant une poignée d'instrument (45') et un corps allongé coulissant en forme de tige (12'; 12") pouvant être actionné par la poignée d'instrument (45'), **caractérisé par** un corps allongé coulissant en forme de tige (12'; 12"') selon l'une des revendications précédentes.

15. Instrument chirurgical à corps allongé coulissant selon la revendication 14, **caractérisé en ce que** la poignée d'instrument (45') et le corps allongé coulissant en forme de tige (12'; 12'') peuvent être reliés mutuellement de manière amovible.
